# EUROPEAN PATENT SPECIFICATION

(11) **EP 1 751 176 B1**
(45) Date of publication and mention of the grant of the patent: **10.03.2010**
(21) Application number: 05742999.5
(22) Date of filing: 04.05.2005
(51) Int. Cl.: C07K 7/64, A61K 38/12

(54) **CYCLOPEPTIDE DERIVATIVES WITH ANTI-INTEGRIN ACTIVITY**
CYCLOPEPTIDDERIVATE MIT ANTI-INTEGRIN-WIRKUNG
DERIVES CYCLOPEPTIDIQUES PRESENTANT UNE ACTIVITE ANTI-INTEGRINES

(30) Priority: 13.05.2004 IT RM20040239
(43) Date of publication of application: 14.02.2007
(73) Proprietor: SIGMA-TAU Industrie Farmaceutiche Riunite S.p.A., 00144 Roma (IT)
(72) Inventor: DAL POZZO, Alma, I-20133 Miolano (IT); PENCO, Sergio, I-20153 Milano (IT); GIANNINI, Giuseppe, I-00040 Pomezia (IT); TINTI, Maria, Ornella, I-00040 Pomezia RM (IT); PISANO, Claudio, I-00040 Pomezia (IT); VESCI, Loredana, I-00040 Pomezia (IT); NI MING HONG, I-20133 Milano (IT)
(74) Representative: Spadaro, Marco
(86) International application number: PCT/IT2005/000262
(87) International publication number: WO 2005/111064

(56) References cited:
- DE-A1- 19 725 368
- HAUBNER R ET AL: "Structural and functional aspects of RGD-containing cyclic pentapeptides as highly potent and selective integrin alphavbeta3 antagonists" JOURNAL OF THE AMERICAN CHEMICAL SOCIETY, AMERICAN CHEMICAL SOCIETY, WASHINGTON, DC, US, vol. 118, 1996, pages 7461-7472, XP002312621 ISSN: 0002-7863

## Description

### Field of the invention

The objects of the present invention are compounds useful as medicaments, processes for the preparation thereof, pharmaceutical compositions containing them and uses thereof for the preparation of medicaments useful for the therapy of diseases due to abnormal angiogenesis.

### Background to the invention

In oncological patients, chemotherapy is, in many cases, the only treatment option for disseminated cancer. One approach to improve the efficacy and reduce the toxicity of anticancer chemotherapy is to administer drugs that target the receptors involved in tumour angiogenesis.

Integrins are involved in the adhesion between one cell and the other and between cells and the extracellular matrix both in the process of tumour angiogenesis and in the metastatic process. In particular, αᵥβ₃ and αᵥβ₅ integrin receptors are strongly expressed in the endothelial cells of human tumour microvessels and in the tumour cells themselves.

The tumour vascularisation is now generally recognised as being a promising target for anticancer therapy [H. Jin and J. Varner, Br. J. Cancer, 2004, 90(3): 561-5].

In recent years, numerous studies have demonstrated that cyclopeptide derivatives containing the Arg-Gly-Asp sequence present high affinity for integrin receptors.

Inhibition of tumor progression and neoangiogenesis using cyclic RGD-peptides is being investigated and some studies have already shown promising results.

For example it has recently been demonstrated in a chemically induced colon carcinoma in rats that late onset of treatment with integrin-blocking peptides resulted in an inhibition of tumour growth and a reduced tumour load which appeared to be mediated, at least in part, by inhibition of neoangiogenesis (Haier J. et al, Clin Exp Metastasis. 2002;19(8):665-72). Therefore αᵥβ₃-integrin-receptor inhibition appears to be a good therapeutic strategy for cancer.

Moreover these cyclic RGD peptides may also have interesting therapeutical applications in cardiovascular diseases, osteoporosis and viral infections utilising cellular integrins for their penetration into the target cells (e.g. HIV). Such compounds are useful for directing chemotherapy drugs, particularly anticancer drugs, against those cells that express high levels of integrin receptors. In this way, an efficacious therapeutic response is achieved with reduced side effects induced by the chemotherapy agent.

Haubner R., et al., J. Am. Chem. Soc., 1996, 118, 7461 studied the influence of conformational changes of RGD-containing cyclic pentapeptides of formula RGD-XX on the inhibition profile of the latter on various types of integrin receptors. By varying the nature of the last two amino acid residues (i.e., XX), the authors concluded that the nature of the last residue did not have any influence on the inhibitory profile of the whole cycle. However, no data regarding αvβ5 were reported.

DE19725368 disclosed derivatives of formula RGD-XX, however, since no biological data regarding such derivative was disclosed in this patent, this document does not give any hindsight on the influence of the various residues at position 4 and/or 5 of the cyclic structure.

### Summary of the invention

The present invention relates to cyclopeptide derivatives endowed with anti-integrin activity, and particularly to cyclic peptides containing, in addition to a sequence of three amino acids which is constant in all the compounds described herein, other two residues consisting in natural and non-natural amino acids, that can be substituted on the nitrogen or on the Cα with a residue consisting in a functional group, or in a terminal chain with a functional group that unexpectedly enhances its binding to integrins αᵥβ₃ and αᵥβ₅. The present invention also relates to processes for the preparation of said compounds, the use thereof as medicaments, particularly as integrin receptor inhibitors, with an action useful in the treatment of diseases such as retinopathy, acute renal failure, osteoporosis and metastases and to pharmaceutical compositions containing them. These compounds, when suitably labelled, are also useful as diagnostic agents for the detection of small tumour masses and arterial occlusion events.

The drugs vehicled by the cyclopeptides according to the present invention belong to cytotoxic agent classes such as alkylating agents (cyclophosphamides, nitrosoureas), antimetabolites (methotrexate, 5-fluorouracyl, cytosine arabinoside), natural products (doxorubicin and structural analogues, actinomycin D, bleomycin, vinca alkaloids, epipodophyllotoxins, and mitomycin C).

For an exhaustive description of the state of the art regarding the αᵥβ₃ and αᵥβ₅ integrin receptor compounds and their applications, the reader is referred to WO 2004/011487, in the name of the Applicant, to which explicit reference is made also in connection with the scientific background.

Surprisingly, the resultant molecules of the present invention demonstrate affinity for integrins, which is sometimes considerably greater than that observed for the cyclopeptides belonging to the same class and described in the literature [H. Kessler, et al., J. Med. Chem., 1999, 42, 3033-40].

Therefore, this invention provides integrin inhibitors of the αᵥβ₃ and αᵥβ₅ type, which are much more potent than the known compounds.

Therefore, the main object of the present invention are compounds of Formula I, as follows:

c(R₁-Arg-Gly-Asp-R₂) (Formula I)

where:
- c means cyclic;
- R₁ an amino acid with general formula: -NX-CY(Z)-CO-; where
- X is selected from the group consisting of: H, linear or branched C₁-C₆ alkyl, C₆-C₁₀ aryl, benzyl, (CH₂)ₙ-COR, (CH₂)ₙ-NHR', 4-COR-benzyl, 4-(CH₂-NHR')-benzyl; where n is an integer number from 1 to 5;
- Y is selected from the group consisting of: H, CHₘF_{m'}; where m + m' = 3, in which m and m' are integer numbers from 0 to 3;
- Z is selected from the group consisting of: H, (CH₂)ₙ₁-NHR', 4-NHR'-(CH₂)ₙ₁-benzyl, 4-COR-benzyl, where n₁ is an integer number from 0 to 5;
- R is selected from the group consisting of: W, OW, N[CH₂-CO-NH-CH₂-O-(CH₂CH₂O)ₙ₂-CH₂-COOW]₂, NH-CH₂-O-(CH₂CH₂O)ₙ₂-CH₂-COOW, NW-(CH₂-CH₂NH)ₙ₂-CH₂-CH₂NHW; in which n₂ is an integer number from 1 to 22; and
- W is selected from the group consisting of: H, C₁-C₃ alkyl;
- R' is selected from the group consisting of: H, CO-(CH₂)ₙ₂-COOW, CO-CH₂-O-(CH₂CH₂O)ₙ₂-CH₂-NHW, CO-CH₂-O-(CH₂CH₂O)ₙ₂-CH₂-COOW; where n₂ has the meaning reported above; and
- R₂ is selected from the group consisting of: D-Phe, D-4-tert-butyl-Phe, D-4-CF3-Phe, D-4-acetylamine-Phe;
their racemic mixtures, their single enantiomers, their single diastereoisomers, and their pharmaceutically acceptable salts.

Preferred examples of formula (I) compounds are:
c(Arg-GIy-Asp-D-Phe-Amp);
c[Arg-Gly-Asp-D-Phe-Aad);
c(Arg-Gly-Asp-D-Phe-N-Me-Amp);
c.[Arg-Gly-Asp-D-Phe-Amp-CO(CH₂)₂COOH];
c(Arg-Gly-Asp-D-Phe-N-Amb-Gly);
c[Arg-Gly-Asp-D-Phe-Amp-(CO-CH₂-(O-CH₂-CH₂)₂-O-CH₂-COOH];
c[Arg-Gly-Asp-D-Phe-Amp-(CO-CH₂-(OCH₂CH₂)₈-OCH₂-COOH]; and
c[Arg-Gly-Asp-D-Phe-Amp(CO-CH₂-(OCH₂-CH₂)₃OCH₃)].

What is meant by pharmaceutically acceptable salt is any salt that does not give rise to toxic or side effects.

Such salts are well known to pharmacologists and to experts in pharmaceutical technology.

The compounds of Formula I may be prepared according to the process described here below and exemplified for the preferred compounds according to the invention. This process constitutes a further object of the invention.

The compounds of Formula I can be prepared, after synthesising the non-natural amino acid residues, according to the conventional techniques of peptide synthesis, as described in the examples in the experimental part. The peptide synthesis can be accomplished either in the solid phase or in solution. Once the suitably protected linear peptide has been prepared, it is cyclised.

The compounds described in the present invention are integrin inhibitors and therefore are useful as medicaments in the treatment of cancer, as diagnostic imaging agents and as targeted drug vectors (G.C. Tucker 2003 Curr. Opin. Investig. Drugs 4, 722-31), particularly for the treatment of tumours whose cells overexpress integrins both naturally and in an induced manner, for example, as a result of radiotherapy; in inflammatory diseases, (e.g. rheumatoid arthritis), in the diseases underlying abnormal angiogenesis, such as tumours, retinopathy, eye diseases, acute renal failure, osteoporosis and metastasis, cardiovascular diseases (stroke and heart damage), and restenosis after percutaneous transluminal coronary angioplasty (J.S. Kerr et al. Drug News Perspect 2001, 14, 143 50). The compounds described herein are also useful, when suitably labelled, as diagnostic agents, especially for the detection of small tumour masses and arterial occlusion events. Various antagonists have been labelled with (18)F, (111)In, (99)Tc, (90)Y and many iodine isotopes, to monitor tumour-induced angiogenesis, since integrins are involved in the migration of endothelial cells for the formation of new vessels (R.H. Haubner et al. 2003 Q. J. Nucl. Med. 47 189-99). High-affinity radiolabelled peptides can be used as targets for αᵥβ₃ integrins and in order to image the areas of vascular damage, inasmuch as αᵥβ₃ integrin expression is increased in the activated endothelial cells and in the vascular smooth muscle cells after vascular damage. This approach overcomes the shortcomings of the magnetic resonance and computed axial tomography imaging methods such as the lack of biologically relevant ligands and blood contrast agents for imaging (F.G. Blankenberg et al. 2002 Am. J. Cardiov. Drugs 2, 357-65).

The pharmaceutical compositions contain at least one compound of Formula I as the active ingredient in an amount such as to produce a significant therapeutic effect. The compositions according to the present invention are entirely conventional and are obtained using methods which are common practice in the pharmaceutical industry. According to the administration route selected, the compositions will be in solid or liquid form, suitable for oral, parenteral or intravenous administration. The compositions according to the present invention contain, along with the active ingredient, at least one pharmaceutically acceptable vehicle or excipient. Particularly useful may be formulation adjuvants, such as, for example, solubilising agents, dispersing agents, suspension agents and emulsifying agents.

The compounds of Formula I can also be used in combination with other anticancer drugs or with other drugs with antiparasite or antiviral activity, both in separate forms or in single dosage form.

The medicaments which are the object of the present invention are also used in the treatment of parasite and adenovirus diseases. Entry of the pathogens into the cells occurs by means of direct penetration of the plasma membrane, clathrin-mediated endocytosis, caveolar endocytosis, pinocytosis or macropinocytosis. Macropinocytosis requires the involvement of integrins (O. Meier et al., 2003, J. Gene Med. 5, 451-62). The antiparasite activity can be exerted then by inhibition of integrin-mediated adhesion and by recruitment of leukocytes guided by the chemokine receptors, e.g. in the control of inflammation induced by *Trypanosoma cruzi*. Incidentally, in the acute phase of Chagas disease, induction of the inflammatory process is crucial for the control of *Trypanosoma cruzi* in the target tissue of the host/parasite equilibrium (J. Lannes-Vieira, 2003, Mem. Inst. Oswaldo Cruz, 98, 299-304).

The following examples further illustrate the invention.

The abbreviations used are:
- Aad: (aminoadipic acid);
- Amb: (aminomethylbenzyl);
- Amp: (aminomethylphenylalanine);
- Boc: (ter-butoxycarbonyl);
- CSA: (camphosulphonic acid);
- CTH: (catalytic transfer hydrogenation);
- DBU: (1,8-Diazabicyclo[5.4.0]undec-7-ene);
- DCC: (dicyclohexylcarbodiimide);
- DCM: (dichloromethane);
- DEAD: (diethyl acetylenedicarboxylate)
- DIEA: (diisopropylethylamine);
- DMF: (dimethylformamide);
- EMEM: (Eagle's minimal essential medium with Earle's salt);
- Fm: (fluorenylmethyl);
- Fmoc: (9-ffuorenylmethyl-oxycarbonyl);
- HOBT: (hydroxybenzotriazole);
- NMP: (N-methyl-pyrrolidone);
- oNBS: (2-nitrobenzenesulfonate);
- PBS: (phosphate buffered saline);
- Pht: (phthaloyl);
- Pmc: (pentamethylchroman-6-sulphonyl);
- SDS: (Sodium dodecylsulfate);
- TBTU: (tetraffuoroborate-O-benzotriazol-1-yl-tetramethyluronium);
- TEA: (triethylamine);
- Teg: (triethyleneglycol monomethylether); and
- TFA: (trifluoroacetic acid);

### Examples

### Example 1

### Synthesis of c(Arg-Gly-Asp-D-Phe-Amp) - ST2581

1.587 mmol of Fmoc-Gly-Res (Res = Sasrin Resin^{®}, Bachem) were suspended under stirring in 75 ml of DMF for 30 minutes, after which 18 ml of piperidine were added, continuing the stirring for a further 30 minutes. The resin, filtered and washed with DMF, was suspended in 50 ml of NMP (N-methyl-pyrrolidone) for 15 minutes, after which Fmoc-Arg(Pmc)-OH, HOBT, TBTU and DIEA were added (3.174 mmol of each); after 2 hours of stirring, the suspension was filtered and washed with DMF. After deprotection with piperidine, the condensation was repeated with the other amino acids in succession, operating each time as described above, namely: Fmoc-Amp(Cbz)-OH, Fmoc-D-Phe-OH, and Fmoc-Asp(OtBu)-OH. After the last deprotection of the Fmoc-N-terminal, the linear pentapeptide is released from the resin with 45 ml of 1% TFA in DCM. This is dissolved in approximately 11 of CH₃CN, and 4.761 mmol of HOBT and TBTU, and 10 ml of DIEA are added; the solution is left to stir for 30 minutes, the solvent is evaporated to a small volume and the precipitation of the product is completed with water.

The filtered crude product was dissolved in thioanisol (50 eq) and TFA (270 eq) and left to stir overnight at room temperature.

The reaction mixture was brought to dryness and the residue taken up with the minimum amount of TFA and re-precipitated with excess ethyl ether. Finally, the crude product was purified by RP-HPLC [Column: Alltima C-18, Alltech; mobile phase: 17% CH₃CN in water + 0.1% TFA].

Analytical HPLC: column: Purosphere STAR, Merck; mobile phase: 15% CH₃CN in water + 0.1% TFA): Rt = 12.15 min.
Molecular mass = 652

### Example 2

### Synthesis of c[Arg-Gly-Asp-D-Phe-Aad) - ST2650

0.69 mmol of Fmoc-Gly-Res were treated exactly as described in example 1, with the difference that in this case the third and fourth amino acids were added in the form of dipeptide Fmoc-D-Phe-Aad(OBzl)-OH. After deprotection of the benzylester by means of CTH, and purification of the crude product with preparatory RP-HPLC (mobile phase: CH₃CN 55% in water + TFA 0.1%; Rt = 17.29 minutes), 187 mg of pure deprotected peptide were obtained. This was dissolved in TFA and, after 1 hour at room temperature, the solution was brought to dryness. The residue was re-dissolved in the minimum amount of TFA and precipitated with excess ethyl ether. The operation was repeated until the clean final product was obtained.

Analytical RP-HPLC (17% CH₃CN in water +0.1% TFA), Rt = 12.52 min.
Molecular mass = 619

### Example 3

### Synthesis of c(Arg-Gly-Asp-D-Phe-N-Me-Amp) - ST2700

To a suspension of Fmoc-Phe(4-Pht-N-CH2)-COOH in anhydrous toluene brought to reflux 2 eq of CSA and 20 eq of paraformaldehyde divided into 4 portions at intervals of 15 minutes were added. The mixture was allowed to cool, diluted with 120 ml of toluene and washed with 5% NaHCO₃ and water. After evaporation of the solvent, the residue was dissolved in 15 ml of CHCl₃ + 15 ml of TFA + 700 µl of Et₃SiH; the mixture was left in the dark to stir for 42 hours. After evaporation of the solvent, the residue was purified by filtration on silica gel. Overall yield: 90%.

The linear peptide was synthesized in solid phase as described in Example 1, inserting Fmoc-N-Me-Phe-(4-Pht-N-CH₂)-COOH as the third amino acid, prepared as described above. In this case the deprotections of N-Fmoc-terminal on resin were carried out with 30% diisopropylamine (300 eq) in DMF solution (due to the presence of phthalimide). After cyclisation, 500 mg of the peptide were dissolved by heating in 10 ml of absolute EtOH, to which 0.9 ml of a solution of NH₂-NH₂·H₂O 1M in ethanol were added. After heating at reflux for 2 hours, the solvent was evaporated and the residue taken up with 10 ml of DCM + 10 ml of Na₂CO₃ solution with vigorous shaking. After evaporation of the organic phase, the crude residue was purified by preparatory RP-HPLC (mobile phase: 17% CH₃CN in water + 0.1% TFA).

Analytical RP-HPLC (16% CH₃CN in water + 0.1% TFA), Rt = 11.7 min
Molecular mass = 665

### Example 4

### Synthesis of c[Arg-Gly-Asp-D-Phe-Amp-(CH₂)₂COOH] - ST2649

120 mg of cyclopeptide c[Arg(Pmc)-Gly-Asp(OtBu)-D-Phe-Amp]·TFA (prepared as described in example 1) were dissolved in 3.6 ml of a mixture of DCM-DMF 2:1, together with a stoichiometric amount of TEA and succinic anhydride. After 1 hour the reaction mixture was diluted with 30 ml of DCM and washed with water. The organic phase, dried and concentrated, yielded a residue of 100 mg of hemisuccinate. This product was completely deprotected with TFA and then submitted to a first purification, as already described in the examples above. It was then further purified by preparatory RP-HPLC (23% CH₃CN in water +0.1%TFA).
Analytical RP-HPLC: (20% CH₃CN in water + 0.1% TFA), Rt = 14.66 min.
Molecular mass = 751

### Example 5

### Synthesis of c(Arg-Gly-Asp-D-Phe-N-Amb-Gly) - ST2701

To a solution of 1.22 mmol of Boc-monoprotected p-xylylenediamine in 6 ml of THF were added 1.83 mmol of TEA and, dropwise, a solution of 1.22 mmol of benzyl bromoacetate in 2 ml of THF. The mixture was left to stir overnight, after which the solvent was evaporated and the residue purified on a flash chromatography column (CHCl₃-EtOAc, 9:1). 0.69 mmol of N-(4-Boc-NH-CH₂-benzyl)-glycine benzylester were obtained.

250 mg of Fmoc-D-Phe-OH were dissolved in 27 ml of DCM and 40 µl of diphosgene and 230 µl of sym-collidine were added; after 15 minutes 190 mg of the previously prepared ester were added, dissolved in 3 ml of DCM. After 3 hours, 80 µl of N-Me-piperazine were added to the reaction mixture and stirred for 10 minutes, after which the mixture was diluted with 10 ml of DCM and extraction was done with water, HCl 0.5 N, water, 5% NaHCO₃ and water. After evaporation of the solvent, the residue was purified by flash chromatography on silica gel (DCM-EtOAc, 9:1). Yield: 80%.

To 100 mg of the product thus obtained, dissolved in 6 ml of MeOH, were added 76 µl of AcOH and 42 mg of HCOONH₄, and the mixture cooled to 0°C, and 50 mg of 10% Pd/C were added. After 30 minutes, the reaction mixture was filtered on celite. The filtrate was brought to dryness and purified on a flash chromatography column (CHCl₃-MeOH 9:1). Yield: 90%.

190 mg of the product thus obtained were dissolved in 1.2 ml of TFA and brought to dryness (deprotection of Boc); the residue was re-dissolved in 9 ml of 10% Na₂CO₃ + 6 ml of dioxane, cooled to 0°C and a solution of 120 µl of benzyloxycarbonyl chloride diluted with 3 ml of dioxane was added dropwise. After 1 hour of stirring at room temperature, evaporation was carried out under vacuum to a small volume, after which the mixture was diluted with water, the pH was reduced to 1 with HCl and extraction was done with EtOAc. After evaporation of the solvent, the residue was purified by filtration on silica gel, washing with CHCl₃-MeOH 8:2). Pure dipeptide yield: 82%.

0.69 mmol of Fmoc-Gly-Res were treated as described in example 1. After Arg, the previously prepared dipeptide Fmoc-D-Phe-N(4-Cbz-NH-CH₂-benzyl)-Gly was added in sequence. The crude product was dissolved in thioanisol and TFA and left to stir at room temperature for 4.5hours. The first purification was done as described in the other examples, while the final purification was done with preparatory HPLC (mobile phase: 16% CH₃CN in water + 0.1% TFA).

Analytical RP-HPLC (15% CH₃CN in water + 0.1% TFA), Rt = 7.67 min.
Molecular mass = 652

### Example 6

### Synthesis of c[Arg-Gly-Asp-D-Phe-Amp-(CO-CH₂-(O-CH₂-CH₂)₂-O-CH₂-COOH] - ST2661.

To a solution of 200 mg of c(Arg(Pmc)-Gly-Asp(OtBu)-D-Phe-Amp)·TFA (obtained as described in example 1) in 4 ml of a 3:1 DCM-DMF mixture was added a substantial excess of glycol diacid. DIEA (3 eq) and DCC (2 eq) were added to the same solution. The mixture was left to stir overnight, after which it was diluted with DCM and washed with water. The crude product was recovered by evaporating the organic phase and purified by flash chromatography (mobile phase: CHCl₃-MeOH 7:3 + 1% AcOH); the fractions containing the product were pooled, washed with water, dehydrated and brought to dryness, and yielded a residue of 157 mg of pure product. This was treated with TFA for 1.5 hours and cleaned as described in the other examples, after which the final purification was done by preparatory HPLC (mobile phase: 22% CH₃CN in water +0.1% TFA).

Analytical RP-HPLC: (23% CH₃CN in water + 0.1% TFA);
Rt = 10 min.
Molecular mass = 855

### Example 7

### Synthesis of c[Arg-Gly-Asp-D-Phe-Amp(CO-CH₂-(OCH₂CH₂)₈-OCH₂-COOH] - ST2874

150 mg of the peptide described in example 1 and 110 mg of PEG 600-COOFm (1 eq) + HOAT (1.5 eq) + DIEA (2 eq) were dissolved in 6 ml of a mixture of DCM-DMF (2:1), and the solution cooled to 0°C; 1.5 eq of DCC were added and the mixture was left to stir overnight. After evaporation of the solvent, the residue was purified on a flash chromatography column (step 1: CHCl₃-MeOH, 96:4; step II: CHCl₃-MeOH, 90:10. For the deprotection of the fluorenylmethylester, 36 mg of the ester were dissolved in 1.8 ml CHCl₃, 41 µl (20 eq) of piperidine were added and left for 1 night at room temperature. After evaporation of the solvent, the crude residue was purified by preparatory HPLC (46% CH₃CN in water + 0.1% TFA). The pure product thus obtained was dissolved in TFA and left for 2 hours at room temperature. After reduction to a small volume, the totally deprotected product was precipitated with excess ethyl ether.

Analytical RP-HPLC (26% CH₃CN in water + 0.1% TFA); Rt = 7.89-15.83 min.
Molecular mass: 1119.

### Example 8

### Synthesis of c[Arg-Gly-Asp-D-Phe-Amp(CO-CH₂-(OCH₂CH₂)₃-OCH₃)]-ST2597

This peptide was synthesized by solid phase as described in the Example 1, inserting Fmoc-Amp(CO-CH₂-Teg)-OH as the third amino acid, which was prepared as following:
570 mg of CH₃O(CH₂CH₂O)₃-CH₂-COOH, 473 mg of 2,3,4,5-pentafluorophenol (Pfp) and 207 µl of pyridine were dissolved with 11.4 ml of DCM. To the solution, cooled to OOC, 637 mg of DCC were added and the reaction mixture left under stirring for 1.5 h. After filtration and washing the filtrate with water, 1 N HCl, water, 5% NaHCO₃ and water, the organic solution was taken to dryness, giving 984 mg of the raw ester.

To a suspension of 500 mg of Fmoc-aminomethylphenylalanine. TFA salt in 15 ml of DCM, 260 µl of TEA was added followed by 800 mg of the activated ester and the mixture left under stirring for 3 h. The crude product was purified by flash chromatography, affording the pure building block.

The final cyclic peptide was purified as usual and isolated from preparative HPLC (27% CH₃CN in water + 1% TFA), Rt= 12.7 min.
Molecular mass = 855

### Example 9

### Biological results

### Binding to integrin αᵥβ₃ receptors

The purified αᵥβ₃ receptor (Chemicon, cat. CC1020) was diluted in buffer (20 mM Tris, pH 7.4, 150 mM NaCl, 2 mM CaCl₂, 1 mM MgCl₂, 1 mM MnCl₂) to a concentration of 0.5 µg/ml. An aliquot of 100 µl was added to 96-well plates and incubated overnight at +4°C. Plates were washed once with buffer (50 mM Tris, pH 7,4, 100 mM NaCl, 2 mM CaCl₂, 1 mM MgCl₂, 1 mM MnCl₂, 1% bovine serum albumin) and then incubated for another 2 hours at room temperature. Plates were washed twice with the same buffer and incubated for 3 hours at room temperature with the radioactive ligand [¹²⁵I[echistatin (Amersham Pharmacia Biotech) 0.05 nM in the presence of competition ligands. At the end of incubation, the wells were washed and the radioactivity determined using a gamma counter (Packard). Non-specific binding of the ligand was determined in the presence of excess cold echistatin (1 µM).

### Binding to integrin αᵥβ₅ receptors

The purified αᵥβ₅ receptor (Chemicon, cat. DCC1020) was diluted in buffer (20 mM Tris, pH 7.4, 150 mM NaCl, 2 mM CaCl₂, 1 mM MgCl₂, 1 mM MnCl₂) to a concentration of 1 µg/ml. An aliquot of 100 µl was added to 96-well plates and incubated overnight at +4°C. Plates were washed once with buffer (50 mM Tris, pH 7.4, 100 mM NaCl, 2 mM CaCl₂, 1 mM MgCl₂, 1 mM MnCl₂, 1% bovine serum albumin) and then incubated for another 2 hours at room temperature. Plates were washed twice with the same buffer: and incubated for 3 hours at room temperature with the radioactive ligand [¹²⁵I]echistatin (Amersham Pharmacia Biotech) 0.15 nM in the presence of competition ligands. At the end of incubation, the wells were washed and the radioactivity determined using a gamma counter (Packard). Non-specific ligand binding was determined in the presence of excess cold echistatin (1 µM).

### Evaluation of IC₅₀ parameters

The affinity of the products for vitronectin receptors was expressed as IC₅₀ value ± SD, i.e. as the concentration capable of inhibiting 50% of the specific radioligand-receptor binding. The IC₅₀ parameter was elaborated using "ALLFIT" software.

### Results

All the RGD peptides examined showed significant affinity for αᵥβ₃ and αᵥβ₅ integrin receptors with an IC₅₀ value of the order of nanomoles. In particular, the most active in inhibiting echistatin binding to the αᵥβ₃ integrins was ST2581 (IC₅₀ = 1.7 nM) followed by the products ST2661 and ST2700 (IC₅₀ = 4 and 7 nM), while the most active for the αᵥβ₅ integrin receptors was the product ST2650 (IC₅₀ = 0.17 nM) followed by the molecules ST2661 and ST2700 (IC₅₀ = 0.35 and 0.99 nM, respectively).

Although the main function of integrins is to mediate cellular adhesion to ECM proteins in intercellular spaces and basement membranes, they also transduce intracellular signals that promote cell migration as well as survival. Integrins have no intrinsic enzymatic activity but activate signaling pathways by coclustering with kinases and adaptor proteins in focal adhesion complexes after their association with polyvalent extracellular matrix (ECM) proteins. For example, integrin ligation suppresses apoptosis by activating suppressors of apoptosis and by inhibitin caspase activation. Integrin also stimulate cell migration by activating Rho and Rac GTPases (guanosine triphosphatases) and by anchoring actin filaments to the membrane. These adhesion proteins promote cell cycle entry by stimulating expression of cyclins. Integrin ligation, therefore, supports signal transduction cascades that promote cell proliferation, survival and migration. In contrast, inhibition of cell integrin-ligand interaction, inhibits cell migration and proliferation and induces apoptosis (Jin H. and Varner J. 2004 Br. J. Cancer 90, 561-565).

**Table 1**

| Affinity of RGD peptides for vitronectin αᵥβ₃ and αᵥβ₅ receptors | | |
|---|---|---|
| **Compound** | **αᵥβ₃** | **αᵥβ₅** |
| | IC₅₀ ± SD (nM) | |
| ST2581 | 1.7±0.1 | 3.4±0.1 |
| ST2597 | 13.5±0.8 | 2.1±0.07 |
| ST2650 | 28.6±0.7 | 0.17±0.01 |
| ST2649 | 37.6±0.9 | 5.1±0.07 |
| ST2661 | 4.0±0.1 | 0.35±0.09 |
| ST2700 | 7.2±0.07 | 0.99±0.005 |
| ST2701 | 36.7±0.7 | 2.9±0.1 |
| ST2874 | 59±0.7 | 712±8.7 |
| Cilengitide | 18.9±3.1 (2) | 0.13±0.009 |

### Adhesion assay of tumor cells on vitronectin

A2780 human ovarian carcinoma and PC3 prostrate carcinoma cells were grown in RPMI 1640 containing 10% fetal bovine serum and 50 µg/ml gentamycin sulfate. A498 human renal carcinoma were grown in EMEM containing 10% fetal bovine serum and 50 µg/ml gentamycin sulfate. All the cells were maintained in a 37°C incubator with saturated humidity and an atmosphere of 95% air and 5% CO₂.

A2780 cell line expresses high levels of αᵥβ₅ integrins, A498 high levels of αᵥβ₃ integrins, and PC3 low levels of both integrins.

To test the effect of the drugs on cell adhesion, the appropriate cellular density (40000-50000 cells/well) for each tumor cell line was incubated with different concentrations of the compounds in 96-well tissue culture plates coated with vitronectin (5 µg/ml) and was allowed to attach for 3 hours. After this time the cells were washed once with PBS containing Ca²⁺ e Mg²⁺. Tumor cells were fixed with 4% paraformaldehyde for 10 min at room temperature and stained with 1% toluidine blue for 10 min at room temperature. Tumor cells were washed with bidistilled water, dried and solubilized with 1% SDS. The number of adherent cells was determined in a microplate reader (Victor2, EG&G Wallac) at 600 nm.

An IC₅₀ value as parameter to measure the inhibiting effect of the molecules on tumor cell adhesion to vitronectin was evaluated using "ALLFIT" computer program. The results obtained with the tested compounds according to the invention are reported in Table 2.

**Table 2**

| Adhesion assay | | | |
|---|---|---|---|
| | **PC3** **IC₅₀, µM** **3 h of treatm.** | **A498** **IC₅₀, µM** **3 h of treatm.** | **A2780** **IC₅₀, µM** **3 h of treatm.** |
| **ST2581** | 21.5±4.3 | 4,3±0,3 | 3,8±0,3 |
| **ST2650** | 38,8±8,5 | 6,5±0,6 | 5,2±0,2 |
| **5T2649** | 33,7±7,8 | 34±2,2 | 9,5±1,9 |
| **ST2661** | 18,8±4,4 | 10,7±0,4 | 12,3±0,9 |
| **5T2700** | 11,1±3,1 | 2,8±0,1 | 0,9±0,1 |
| **ST2701** | 22,7±5,1 | 9,0±0,2 | 3,2±0,4 |

## Claims

1. Cyclic peptides having the following Formula I:
c(R₁-Arg-Gly-Asp-R₂) (Formula I)
where:
c means cyclic;
R₁ an amino acid with general formula: -NX-CY(Z)-CO-; where
X is selected from the group consisting of: H, linear or branched C₁-C₆ alkyl, C₆-C₁₀ aryl, benzyl, (CH₂)ₙ-COR, (CH₂)ₙ-NHR', 4-COR-benzyl, 4-(CH₂-NHR')-benzyl; where n is an integer number from 1 to 5;
Y is selected from the group consisting of: H, CHₘF_{m';} where m + m' = 3, in which m and m' are integer numbers from 0 to 3;
Z is selected from the group consisting of: H, (CH₂)ₙ₁-NHR', 4-NHR'-(CH₂)ₙ₁-benzyl, 4-COR-benzyl, where n₁ is an integer number from 0 to 5;
R is selected from the group consisting of: W, OW, N[CH₂-CO-NH-CH₂-O-(CH₂CH₂O)ₙ₂-CH₂-COOW]₂, NH-CH₂-O-(CH₂CH₂O)ₙ₂-CH₂-COOW, NW-(CH₂-CH₂NH)ₙ₂-CH₂-CH₂NHW; in which n₂ is an integer number from 1 to 22; and
W is selected from the group consisting of: H, C₁-C₃ alkyl;
R' is selected from the group consisting of: H, CO-(CH₂)ₙ₂-COOW, CO-CH₂-O-(CH₂CH₂O)ₙ₂-CH₂-NHW, CO-CH₂-O-(CH₂CH₂O)ₙ₂-CH₂-COOW; where n₂ has the meaning reported above; and
R₂ is selected from the group consisting of: D-Phe, D-4-tert-butyl-Phe, D-4-CF₃-Phe, D-4-acetylamine-Phe;
their racemic mixtures, their single enantiomers, their single diastereoisomers, and their pharmaceutically acceptable salts.

2. A compound according to claim 1 selected from the group consisting of:
c(Arg-Gly-Asp-D-Phe-Amp);
c(Arg-Gly-Asp-D-Phe-N-Me-Amp);
c[Arg-Gly-Asp-D.Phe-Amp-CO(CH₂)₂COOH];
c(Arg-Gly-Asp-D-Phe-N-Amb-Gly);
c[Arg-Gly-Asp-D-Phe-Amp-(CO-CH₂-(O-CH₂-CH₂)₂-O-CH₂-COOH];
c[Arg-Gly-Asp-D-Phe-Amp-(CO-CH₂-(OCH₂CH₂)₈-OCH₂-COOH].

3. Compound of formula:
c[Arg-Gly-Asp-D-Phe-Amp(CO-CH₂-(OCH₂-CH₂)₃0CH₃)];
c[Arg-Gly-Asp-D-Phe-Aad).

4. Process for the preparation of the compounds according to claims 1-3 comprising the synthesis of the linear peptide and its subsequent cyclisation.

5. Process according to claim 4, in which the synthesis of the peptide is accomplished in the solid phase or in solution.

6. Use of compounds according to claims 1-3 for the preparation of medicaments.

7. Pharmaceutical compositions containing at least one compound according to claim 1 or 2 or 3 in mixtures with at least one pharmaceutically acceptable excipient or vehicle.

8. Compositions according to claim 7 additionally containing a drug selected from the group consisting of anticancer, antiparasite or antiviral agents, either in separate forms or in single dosage form.

9. Use of compounds according to claims 1-3 for the preparation of a medicament with integrin receptor inhibiting activity.

10. Use according to claim 9, in which said medicament is useful for the treatment of diseases deriving from abnormal angiogenesis.

11. Use according to claim 10, in which said disease is selected from the group consisting of tumours that overexpress integrins both naturally and in an induced manner, inflammatory forms (e. g. rheumatoid arthritis), eye diseases, retinopathy, acute renal failure, osteo-porosis and metastases, cardiovascular diseases (stroke and heart damage).

12. Use of compounds according to claims 1-3 for the preparation of a medicament with antiparasite activity through integrin inhibition.

13. Composition containing a radiolabelled derivative of a compound according to either of claims 1 or 2 or 3.

14. Use of a radiolabelled derivative of a compound according to either of claims 1 or 2 or 3 for the preparation of a diagnostic agent.

15. Use according to claim 14, in which said diagnostic agent is used for the detection of small tumour masses or arterial occlusion events.

## Patentansprüche

1. Cyclische Peptide, welche die folgende Formel I besitzen:
c (R₁-Arg-Gly-Asp-R₂) (Formel I)
wo:
c cyclisch bedeutet;
R₁ eine Aminosäure mit der allgemeinen Formel: -NX-CY(Z)-CO-; wo
X ausgewählt ist aus der Gruppe, bestehend aus: H, linearem oder verzweigtem C₁-C₆-Alkyl, C₆-C₁₀-Aryl, Benzyl, (CH₂)ₙ-COR, (CH₂)ₙ-NHR', 4-COR-Benzyl, 4-(CH₂-NHR')-Benzyl; wo n eine ganze Zahl von 1 bis 5 ist;
Y ausgewählt ist aus der Gruppe, bestehend aus: H, CHₘF_{m'}; wo m + m' = 3, worin m und m' ganze Zahlen von 0 bis 3 sind;
Z ausgewählt ist aus der Gruppe, bestehend aus: H, (CH₂)ₙ₁-NHR', 4-NHR'-(CH₂)ₙ₁-Benzyl, 4-COR-Benzyl, wo n1 eine ganze Zahl von 0 bis 5 ist;
R ausgewählt ist aus der Gruppe, bestehend aus:
W, OW, N[CH₂-CO-NH-CH₂-O-(CH₂CH₂O)ₙ₂-CH₂-COOW]₂, NH-CH₂-O-(CH₂CH₂O)ₙ₂-CH₂-COOW, NW-(CH₂-CH₂NH)ₙ₂-CH₂-CH₂NHW; worin n2 eine ganze Zahl von 1 bis 22 ist; und
W ausgewählt ist aus der Gruppe, bestehend aus: H, C₁-C₃-Alkyl;
R' ausgewählt ist aus der Gruppe, bestehend aus: H, CO-(CH₂)ₙ₂-COOW, CO-CH₂-O-(CH₂CH₂O)n₂-CH₂-NHW, CO-CH₂-O-(CH₂CH₂O)ₙ₂-CH₂-COOW; wo n2 die oben angegebene Bedeutung hat; und
R₂ ausgewählt ist aus der Gruppe, bestehend aus: D-Phe, D-4-tert-Butyl-Phe, D-4-CF₃-Phe, D-4-Acetylamin-Phe;
ihre racemischen Mischungen, ihre einzelnen Enantiomere, ihre einzelnen Diastereomere und ihre pharmazeutisch verträglichen Salze.

2. Verbindung gemäß Anspruch 1, ausgewählt aus der Gruppe, bestehend aus:
c(Arg-Gly-Asp-D-Phe-Amp);
c(Arg-Gly-Asp-D-Phe-N-Me-Amp);
c[Arg-Gly-Asp-D-Phe-Amp-CO(CH₂)₂COOH];
c(Arg-Gly-Asp-D-Phe-N-Amb-Gly);
c[Arg-Gly-Asp-D-Phe-Amp-(CO-CH₂-(O-CH₂-CH₂)₂-O-CH₂-COOH];
c[Arg-Gly-Asp-D-Phe-Amp-(CO-CH₂-(OCH₂CH₂)₈-OCH₂-COOH].

3. Verbindung der Formel:
c[Arg-Gly-Asp-D-Phe-Amp(CO-CH₂-(OCH₂-CH₂)₃OCH₃)];
c[Arg-Gly-Asp-D-Phe-Aad).

4. Verfahren zur Herstellung der Verbindungen gemäß den Ansprüchen 1-3, welches die Synthese des linearen Peptids und dessen anschließende Cyclisierung umfasst.

5. Verfahren gemäß Anspruch 4, in welchem die Synthese des Peptids in fester Phase oder in Lösung durchgeführt wird.

6. Verwendung von Verbindungen gemäß den Ansprüchen 1-3 für die Herstellung von Medikamenten.

7. Pharmazeutische Zusammensetzungen, welche mindestens eine Verbindung gemäß den Ansprüchen 1 oder 2 oder 3 in Mischungen mit wenigstens einem pharmazeutisch verträglichen Hilfsstoff oder Träger enthalten.

8. Zusammensetzungen gemäß Anspruch 7, welche zusätzlich ein Arzneimittel enthalten, das ausgewählt ist aus der Gruppe, bestehend aus Antikrebsmitteln, antiparasiären oder antiviralen Agenzien, entweder in getrennten Formen oder als Einzeldosisform.

9. Verwendung von Verbindungen gemäß den Ansprüchen 1-3 für die Herstellung eines Medikaments mit Integrin-Rezeptor-hemmender Aktivität.

10. Verwendung gemäß Anspruch 9, in welcher das genannte Medikament für die Behandlung von Krankheiten geeignet ist, die von abnormer Angiogenese herrühren.

11. Verwendung gemäß Anspruch 10, in welcher die genannte Krankheit ausgewählt ist aus der Gruppe, bestehend aus Tumoren, welche Integrine, sowohl natürlich als auch auf induziert Weise überexprimieren, Entzündungsformen (z. B. rheumatoide Arthritis), Augenkrankheiten, Retinopathie, akutem Nierenversagen, Osteoporose und Metastasen, kardiovaskulären Krankheiten (Schlaganfall und Herzschaden).

12. Verwendung von Verbindungen gemäß den Ansprüchen 1-3 für die Herstellung eines Medikaments mit antiparasitärer Aktivität durch Integrin-Hemmung.

13. Zusammensetzung, welche ein radiomarkiertes Derivat einer Verbindung gemäß einem der Ansprüche 1 oder 2 oder 3 enthält.

14. Verwendung eines radiomarkierten Derivats einer Verbindung gemäß einem der Ansprüche 1 oder 2 oder 3 für die Herstellung eines diagnostischen Agens.

15. Verwendung gemäß Anspruch 14, in welcher das genannte diagnostische Agens für die Detektion kleiner Tumormassen oder von Arterienverschluss-Ereignissen.

## Revendications

1. Peptides cycliques ayant la formule I suivante :
c (R₁-Arg-Gly-Asp-R₂) (Formule I)
dans laquelle :
c signifie cyclique ;
R₁ est un acide aminé ayant la formule générale : -NX-CY(Z)-CO- ; où
X est choisi dans le groupe constitué par : H, un alkyle en C₁-C₆ linéaire ou ramifié, un aryle en C₆-Cₗ₀, un benzyle, (CH₂)ₙ-COR, (CH₂)ₙ-NHR', 4-COR-benzyle, 4-(CH₂-NHR')-benzyle ; où n est un nombre entier de 1 à 5 ;
Y est choisi dans le groupe constitué par : H, CHₘFₘ' ; où m + m' = 3, où m et m' sont des nombres entiers de 0 à 3 ;
Z est choisi dans le groupe constitué par : H, (CH₂)ₙ₁-NHR', 4-NHR'-(CH₂)ₙ₁-benzyle, 4-COR-benzyle, où n1 est un nombre entier de 0 à 5 ;
R est choisi dans le groupe constitué par : W, OW, N[CH₂-CO-NH-CH₂-O-(CH₂CH₂O)ₙ₂-CH₂-COOW]₂, NH-CH₂-O(CH₂CH₂O)ₙ₂-CH₂-COOW, NW-(CH₂-CH2NH)ₙ₂-CH₂-CH₂NHW; où n2 est un nombre entier de 1 à 22 ; et
W est choisi dans le groupe constitué par : H, un alkyle en C₁-C₃ ;
R' est choisi dans le groupe constitué par : H, CO-(CH₂)ₙ₂-COOW, CO-CH₂-O-(CH₂CH₂O)ₙ₂-CH₂-NHW, CO-CH₂-O-(CH₂CH₂O)ₙ₂-CH₂-COOW; où n2 a la signification présentée ci-dessus ; et
R₂ est choisi dans le groupe constitué par : D-Phe, D-4-tert-butyl-Phe, D-4-CF₃-Phe, D-4-acétylamine-Phe ;
leurs mélanges racémiques, leurs énantiomères uniques, leurs diastéréomères uniques, et leurs sels pharmaceutiquement acceptables.

2. Composé selon la revendication 1, choisi dans le groupe constitué par :
c(Arg-Gly-Asp-D-Phe-Amp);
c(Arg-Gly-Asp-D-Phe-N-Me-Amp);
c[Arg-Gly-Asp-D-Phe-Amp-CO(CH2)₂COOH];
c(Arg-Gly-Asp-D-Phe-N-Amb-Gly);
c[Arg-Gly-Asp-D-Phe-Amp-(CO-CH₂-(O-CH₂-CH₂)₂-O-CH₂-COOH];
c[Arg-Gly-Asp-D-Phe-Amp-(CO-CH₂-(OCH₂CH₂)₈-OCH₂-COOH].

3. Composé de formule :
c)Arg-Gly-Asp-D-Phe-Amp(CO-CH₂-(OCH₂-CH₂)₃OCH₃)];
c[Arg-Gly-Asp-D-Phe-Aad).

4. Procédé pour la préparation des composés selon les revendications 1 à 3, comprenant la synthèse du peptide linéaire et sa cyclisation subséquente.

5. Procédé selon la revendication 4, dans lequel la synthèse du peptide est accomplie en phase solide ou en solution.

6. Utilisation de composés selon les revendications 1 à 3 pour la préparation de médicaments.

7. Compositions pharmaceutiques contenant au moins un composé selon la revendication 1 ou 2 ou 3 en mélanges avec a u moins un excipient ou véhicule pharmaceutiquement acceptable.

8. Compositions selon la revendication 7, contenant de plus une substance médicamenteuse choisie dans le groupe constitué par les agents anticancéreux, antiparasitaires ou antiviraux, soit en formes séparées soit en forme de dosage unique.

9. Utilisation de composés selon les revendications 1 à 3 pour la préparation d'un médicament ayant une activité inhibant les récepteurs d'intégrine.

10. Utilisation selon la revendication 9, dans laquelle ledit médicament est utile pour le traitement de maladies dérivant d'une angiogenèse anormale.

11. Utilisation selon la revendication 10, dans laquelle ladite maladie est choisie dans le groupe constitué par les tumeurs qui surexpriment des intégrines à la fois naturellement et d'une manière induite, les formes inflammatoires (par exemple, polyarthrite rhumatoïde), les maladies des yeux, une rétinopathie, une défaille rénale aiguë, une ostéoporose et les métastases, les maladies cardio-vasculaires (attaque et lésion cardiaque).

12. Utilisation de composés selon les revendications 1 à 3, pour la préparation d'un médicament ayant une activité antiparasitaire par inhibition des intégrines.

13. Composition contenant un dérivé radiomarqué d'un composé selon l'une quelconque des revendications 1 ou 2 ou 3.

14. Utilisation d'un dérivé radiomarqué d'un composé selon l'une quelconque des revendications 1 ou 2 ou 3, pour la préparation d'un agent diagnostique.

15. Utilisation selon la revendication 14, dans laquelle ledit agent diagnostique est utilisé pour la détection de petites masses tumorales ou d'événements d'occlusion artérielle.
